# EUROPEAN PATENT SPECIFICATION

(11) **EP 4 103 768 B1**
(45) Date of publication and mention of the grant of the patent: **15.07.2026**
(21) Application number: 21709098.4
(22) Date of filing: 12.02.2021
(51) Int. Cl.: D01D 5/00, D01F 1/10, D04H 1/728, A61K 8/02, A61K 8/65, A61K 8/73, D01F 2/28, D01F 4/00, D01F 9/00

(54) **COMPOSITION FOR ELECTROSPINNING**
ZUSAMMENSETZUNG FÜR ELEKTROSPINNEN
COMPOSITION POUR ÉLECTROFILAGE

(30) Priority: 13.02.2020 IT 202000002827
(43) Date of publication of application: 21.12.2022
(73) Proprietor: Bakel S.P.A., 33100 Udine (IT)
(72) Inventor: GREGORIS, Raffaella, 33100 Udine (IT); MANFREDINI, Stefano, 44049 Vigarano Mainarda (IT); VERTUANI, Silvia, 44123 Ferrara (IT); ROSO, Martina, 37142 Verona (IT)
(74) Representative: Petraz, Gilberto Luigi
(86) International application number: PCT/IB2021/051188
(87) International publication number: WO 2021/161248

(56) References cited:
- EP-B1- 2 079 860
- CN-A- 107 675 359
- US-A1- 2006 264 130
- QIN ET AL.: "Fast dissolving oral films for drug delivery prepared from chitosan/pullulan electrospinning nanofibers", vol. 137, 15 September 2025 (2025-09-15), pages 224 - 231, Retrieved from the Internet <URL:https://www.sciencedirect.com/science/article/pii/S0141813019339170> [retrieved on 20250519], DOI: https://doi.org/10.1016/j.ijbiomac.2019.06.224
- "Fabrication and characterization of tea polyphenols loaded pullulan-CMC electrospun nanofiber for fruit preservation", 12 October 2017 (2017-10-12), Retrieved from the Internet <URL:https://pubmed.ncbi.nlm.nih.gov/29032085/> [retrieved on 20250519]
- ATILA ET AL.: "Crosslinked pullulan/cellulose acetate fibrous scaffolds for bone tissue engineering", vol. 69, 8 August 2016 (2016-08-08), pages 1103 - 1115, XP029725036, Retrieved from the Internet <URL:https://pubmed.ncbi.nlm.nih.gov/27612808/> [retrieved on 20250519], DOI: 10.1016/j.msec.2016.08.015
- LIU ET AL.: "Electrospun Food-Grade Ultrafine Fibers from Pectin and Pullulan Blends", vol. 7, no. 7, 29 June 2016 (2016-06-29), XP055706253, Retrieved from the Internet <URL:https://www.scirp.org/journal/paperinformation?paperid=67821> [retrieved on 20250519], DOI: 10.4236/fns.2016.77065
- SAQUING ET AL., INDUSTRIAL AND ENGINEERING CHEMISTRY RESEARCH, vol. 52, 23 May 2025 (2025-05-23), pages 8692 - 8704, Retrieved from the Internet <URL:https://cris.technion.ac.il/en/publications/alginate-polyethylene-oxide-blend-nanofibers-and-the-role-of-the-> [retrieved on 20250520], DOI: https://doi.org/10.1021/ie302385b

## Description

### FIELD OF THE INVENTION

The present invention concerns a composition to be electrospun, that is, which allows to produce nanofibers by electrospinning. More particularly, the composition is the type based on a polymer, preferably a biocompatible polymer, to be electrospun.

### BACKGROUND OF THE INVENTION

The electrospinning process is known, which allows to obtain nanofibers, that is, continuous fibers with a diameter in the order of a nanometer, starting from a composition based on a polymer compound to be electrospun that is subjected to an electric field. Depending on the type of compound that is electrospun, the nanofibers obtained can then have applications in any field whatsoever, for example in medicine, military defense, environment, biotechnology, energy or in the cosmetic field.

For environmental reasons, electrospinning tends to be performed using ecological solvents, in particular in water. However, electrospinning polymers in pure water is not simple due to its surface tension and the viscosity of the compound that is obtained.

To overcome this problem, attempts have been made to lower the surface tension and viscosity of the water, for example by electrospinning in aqueous solutions of ammonium, therefore in solutions with a high pH, or also in water in the presence of dimethylformamide DMF at 40°C. Experiments have also been performed with hexafluoroisopropanol HFIP or ethanol. By proceeding in this way, however, the ecological aspect is compromised.

Some alternative solutions have been proposed. For example, US 2006/264130 describes a method to produce nanometric fibers by electrospinning a composition comprising a polysaccharide and an active ingredient. The composition can comprise any polysaccharide or mixture of polysaccharides provided that they are soluble or dispersible in water. This document also describes the addition of ethanol to help the electrospinning of a polysaccharide.

EP2079860 shows a method to produce microtubes or nanotubes by electrospinning two polymer solutions, in which the polymers can be biocompatible. The first polymer solution serves to create the microtube, while the second polymer solution serves to create a coating on the internal surface of the microtube. Each electrospun solution comprises a single polymer to be electrospun.

CN107675359 describes a method to prepare composite fibers of pullulan and sodium alginate by electro spinning. The alginate is used to modulate the viscosity of the liquid solution of pullulan that is electrospun.

The article "Fast dissolving oral films for drug delivery prepared from chitosan/pullulan electrospinning nanofibers" by Qin et al, Science Direct, vol. 137, p. 224-231 discusses the mixing of pullulan with chitosan for the production of nanofibers, and verifies the influence of chitosan addition on electrospun pullulan fibers.

The article "Fabrication and characterization of tea polyphenols loaded pullulan-CMC electrospun nanofiber for fruit presetvation" by Shao et al, International Journal of Biological Macromolecules, vol. 107, p. 1908-1914, describes a pullulan:CMC 90:10 composition for electrospinning, with polyphenols added at different percentages. Some conditions of the electrospinning phase are varied (voltage and feed rate of the composition to the needle) to verify their influence on the morphology of the fibers obtained, in particular on the diameter of the fibers and the probability of accumulation formation. It is also indicated that the concentration of polyphenols has an influence on the diameter of the fibers.

The article, "Crosslinked pullulan/cellulose acetate fibrous scaffolds for bone tissue engineering" by Atila et al, Mater Sci Eng Mater Biol Appl, vol. 69, p. 1103-1115 relates to the formation of scaffolds based on pullulan and cellulose acetate, obtained by electrospinning in organic solvents, in particular DMAc/DMSO, but does not mention electrospinning in aqueous solution.

The article "Electrospun Food-Grade Ultrafine Fibers from Pectin and Pullulan Blends" by Liu et al, Food and Nutrition Sciences, vol. 7, n. 7, concerns the formation of food-grade pectin and pullulan nanofibers for future food applications.

The article "Alginate-Polyethylene Oxide Blend Nanofibers and the Role of the Carrier Polymer in Electrospinning" by Saquing et al, Industrial & Engineering Chemistry Research, vol. 52, p. 8692-8704, is an in-depth study of the alginate:PEO mixture, in particular the authors seek to understand why alginate can be electrospun in water only in the presence of PEO.

However, even these solutions do not fully satisfy the needs of the sector with regard to the surface tension of water as a solvent, and the viscosity of the solution obtained.

There is therefore a need to perfect a composition to be electrospun which can overcome at least one of the disadvantages of the state of the art.

In particular, one purpose of the present invention is to provide a composition to be electrospun that can be electrospun in pure water or aqueous solutions that have no environmental impact.

Another purpose of the present invention is to provide a composition to be electrospun which allows to produce continuous nanofibers, or in any case fibers, with an almost constant diameter and free from defects.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, this application describes a composition to be electrospun, which overcomes the limits of the state of the art and eliminates the defects present therein.

In accordance with some embodiments, the composition comprises hyaluronic acid as a first compound to be electrospun and pullulan or a mixture of alginate:poly(oxyethylene) 1:1 as a spinning promoter. The spinning promoter has the function of facilitating the spinning of the first compound, in particular of establishing the electrospinning method so as to obtain regular fibers.

According to some embodiments, the composition also comprises an active ingredient. This active ingredient can, for example, be selected from peptides, amino acids, antioxidants and vitamins.

According to some embodiments, the composition also comprises a stabilizer, suitable to improve the stability of the fibers obtained following the electrospinning. Preferably, the stabilizer is a cross-linkable polymer.

An advantage of the composition as above lies in the possibility of making a cosmetic product with topical concentrations of, for example, hyaluronic acid much higher than those obtainable with traditional formulations. With the known compositions, it is practically impossible to reach high concentrations, even with hyaluronic acid, polysaccharides, collagen, hydroxypropyl methylcellulose HPMC and their derivatives with low molecular weight, since the viscosity of the product increases too much and it is not possible to exceed 5-10% by weight. With the present composition, it is possible to obtain cosmetic products that allow to apply concentrations up to 50% by weight of hyaluronic acid on the skin.

According to one aspect, there is also provided a method to prepare a composition to be electrospun, in which hyaluronic acid as a first compound to be electrospun and pullulan or a mixture alginate:poly(oxyethylene) 1:1 as a spinning promoter are mixed.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a graphic representation of the evolution of skin hydration with the application of a cosmetic product made with the composition of the invention, and with the application of a placebo product;
- fig. 2 is a graphic representation of the evolution of skin elasticity with the application of a cosmetic product made with the composition of the invention, and with the application of a placebo product;
- fig. 3 is a graphic representation of the evolution of the density of collagen in skin with the application of a cosmetic product made with the composition of the invention, and with the application of a placebo product;
- figs. 4 and 5 are graphic representations of the evolution of the presence of collagen in skin treated with a cosmetic product made with the composition of the invention, and the comparison with untreated skin.

### DETAILED DESCRIPTION OF SOME EMBODIMENTS

We will now refer in detail to the possible embodiments of the invention. Each example is supplied by way of illustration of the invention and shall not be understood as a limitation thereof. For example, one or more characteristics shown or described insomuch as they are part of one embodiment can be varied or adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description. The present description can provide other embodiments and can be obtained or executed in various other ways. We must also clarify that the phraseology and terminology used here is for the purposes of description only, and cannot be considered as limitative.

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

All measurements are carried out, unless otherwise indicated, at 25°C (room temperature) and at atmospheric pressure. All temperatures, unless otherwise indicated, are expressed in degrees Celsius.

All percentages and ratios indicated here are understood to refer to the weight of the total composition (w/w), unless otherwise indicated.

All percentage intervals reported here are supplied with the provision that the sum with respect to the overall composition is 100%, unless otherwise indicated.

All the intervals reported here shall be understood to include the extremes, including those that report an interval "between" two values, unless otherwise indicated.

The present description also includes the intervals that derive from overlapping or uniting two or more intervals described, unless otherwise indicated.

The present description also includes the intervals that can derive from the combination of two or more values taken at different points, unless otherwise indicated.

Where water is mentioned, we mean distilled water, unless otherwise specified.

The composition comprises a first compound to be electrospun.

The first compound to be electrospun is a biocompatible polymer suitable to be electrospun and is selected from a group consisting of polysaccharides, collagen, hydroxypropyl methylcellulose HPMC and their derivatives. Preferably, the first compound to be electrospun is a first polysaccharide to be electrospun, even more preferably selected from hyaluronic acid, starch, amylose, cellulose, chitosan, glycogen, pectin and agar.

In the event the polysaccharide to be electrospun is hyaluronic acid, it can be of the linear or cross-linked type, and can have a high mass, for example in the order of a million Dalton or even more, or alternatively have a low mass, typically in the order of 10,000 Daltons or less. It is also possible to provide a mixture of linear hyaluronic acid with cross-linked hyaluronic acid, so as to modulate the rigidity of the yarn that will be obtained, as well as the three-dimensional structure of a film that can be obtained by depositing the yarn on a support.

The first compound is preferably diluted in an aqueous or aqueous-based solution, at low concentrations, for example between 0.5% and 5% by weight, more preferably between 0.6% and 2.5% by weight.

The composition to be electrospun also comprises a spinning promoter which is a carrier polymer without filler, favorably biocompatible. It is selected from the group consisting of polysaccharides, provided that they are chemically different from the first polysaccharide to be electrospun, possibly combined with poly(oxyethylene) PEO. PEO is a good candidate as a promoter, since it is watersoluble and biocompatible.

Preferably, the promoter polysaccharide can be selected from alginate, possibly in the presence of PEO, and pullulan. Even more preferably, the electrospinning promoter is selected from an alginate:PEO mixture and pullulan.

The alginate and PEO are preferably diluted separately in aqueous or aqueous-based solutions, at a concentration comprised between 2% and 30%, more preferably between 4% and 10% by weight. The alginate:PEO mixture is made in proportions preferably comprised between 5:1 and 1:5, more preferably between 2:1 and 1:2. The best electrospinning results were obtained with proportions equal to 1:1.

Pullulan, on the other hand, is diluted in an aqueous or aqueous-based solution preferably at a concentration comprised between 3% and 30%, more preferably between 10% and 20% by weight. The first compound to be electrospun and the promoter are mixed in proportions (first compound):promoter preferably comprised between 4:1 and 1:7, more preferably between 3:1 and 1:6.

According to some embodiments, the composition also comprises an active ingredient, for example selected from peptides, amino acids, antioxidants and vitamins. These active ingredients are particularly useful, in particular for applications in the medical and/or cosmetic field.

If an active ingredient is present, the first compound to be electrospun can be composed of a mixture of linear hyaluronic acid with cross-linked hyaluronic acid. Cross-linked hyaluronic acid has the effect of increasing the rigidity of the nanometric fibers obtained, but also of increasing the complexity of the three-dimensional structure of a film obtained by means of the continuous deposit of the fibers obtained on several layers. In particular, the presence of cross-linked hyaluronic acid causes the formation of cavities in the film, cavities that allow to house the molecules of active ingredient.

Preferably, the active ingredient is introduced into the mixture of compound to be spun and promoter, before the electrospinning.

According to some embodiments, the composition also comprises a stabilizer, which is preferably a cross-linkable polymer. One example of a stabilizer is sodium alginate, which has to be added to pullulan as a promoter. Preferably, the proportion of pullulan:alginate is comprised between 3:1.5 and 3:0.5, more preferably it is equal to 3:1.

### EXAMPLES

Electrospinning tests were performed on examples of a composition according to the present description. In the composition examples, the first compound to be electrospun is selected from the compounds listed in the table below:

| | |
|---|---|
| HA1 | linear hyaluronic acid with average molecular mass equal to 1.2 MDa |
| HA2 | hyaluronic acid oligomer with average molecular mass lower than 10000 Da |
| HA3 | hyaluronic acid with average molecular mass equal to 50000 Da |
| HA4 | cross-linked hyaluronic acid with average molecular mass comprised between 20 and 3000 kDa |

These compounds are supplied by Esperis S.p.A., Milan, Evonik Degussa Italia, Cremona, and IRALAB S.p.A., Usmate Velate (MI). The molecular masses were determined by means of GPC (Gel Permeation Chromatography).

The electrospinning was performed in a NANON.01A apparatus of the Japanese company Mecc CO. Ltd. The experimental conditions are indicated in each of the examples below.

The fibers produced were characterized by means of scanning electron microscopy. In particular, they were coated with gold using an EMITECHK950x Turbo Evaporator sputter coater, EBSciences, East Granby, CT, and observed with a Cambridge Stereoscan 440 SEM, Cambridge, UK scanning electron microscope.

### Examples of electrospinning of compositions comprising hyaluronic acid as a compound to be electrospun and a PEO:alginate mixture as a spinning promoter

The spinning promoter comprises a mixture of an aqueous solution of alginate at 5% by weight with an aqueous solution of PEO at 5% by weight in a proportion of 1:1. The promoter was then mixed with an aqueous solution of linear hyaluronic acid with an average molecular weight of 1.2 MDa at 0.5% by weight. The promoter:(HA solution) proportion is equal to 5.6:1. The composition was electrospun with relative humidity (RH) comprised between 24% and 29%, at a temperature of 22°C, with an electric field of 20kV, at a volumetric flow rate of the composition at the head equal to 0.7 mL/h, the distance between the spinning head and the support on which the fiber deposits is equal to 15cm and the needle used being a 22G type needle. The fiber obtained is regular and has few defects. The same promoter was mixed with an aqueous solution of hyaluronic acid oligomer at 13% by weight, in promoter:(HA solution) proportion equal to 1:3. The electrospinning of this second example of composition, under the same operating conditions as the first example as above, has a very regular and defect-free fiber, with an average diameter comprised between 250 and 350 nm. The fiber obtained completely covered the support used.

### Examples of electrospinning of compositions comprising hyaluronic acid as a compound to be electrospun and pullulan as a spinning promoter

The pullulan used is of the food grade type, produced by Hayashibara Co., Ltd. Aqueous solutions of pullulan at 10%, 15% or 20% by weight were prepared, and these aqueous solutions of pullulan (spinning promoter) were mixed with aqueous solutions of hyaluronic acid, for the electrospinning.

The table below lists the examples of compositions that were electrospun, as well as the corresponding operating conditions of the electrospinning.

| | Composition | Electrospinning conditions |
|---|---|---|
| 1 | Pullulan 20%:HA3 29% 1:2 | RH 20-30%, 23kV, 0.1µl/min, 15 cm, needle 22G |
| 2 | Pullulan 20%:HA2 29% 1:2 | RH 46%, T=23°C, 23kV, 1ml/h, 15 cm, needle 22G |
| 3 | Pullulan 10%:HA2 23% 1:3 | RH 40%, T=24°C, 23-25kV, 1ml/h, needle 22G, max distance |
| 4 | Pullulan 10%:HA2 15% 1:2 | RH 49%, T=21°C, 23kV, 0.6ml/h, 18 cm, needle 22G, acid pH (between 1.5 and 3) |
| 5 | (pullulan 15%/alginate 5% 3:1):HA2 23% 1:3 | RH 49%, T=21°C, 23kV, 0.6ml/h, 15 cm, needle 22G |
| 6 | Pullulan 10%:HA2 23% 1:3 | RH 49%, T=21°C, 23kV, 0.15ml/h, 15 cm, needle 22G, acid pH (between 1.5 and 3) |
| 7 | Pullulan 10%:HA2 15% 1:2 | RH 40-50%, T=21°C, 23kV, 0.6ml/h, 15 cm, needle 22G, pH = 5.5 |
| 8 | Pullulan 10%:HA2 23% 1:3 | RH 30%, T=22°C, 23kV, 0.5ml/h, 15 |
| | | cm, needle 22G, pH = 5.5 |

Example 1 resulted in regular fibers, without defects and with an average diameter from 400 to 700 nm. However, little deposit was observed during the test.

In example 2, the fibers obtained are thick, with an average diameter of 10 µm, due to the high viscosity of the electrospun solution.

In example 3, the fibers obtained have an average diameter comprised between 50 nm and 2 µm. It should be observed that with this example the fibers were deposited both on aluminum and also on a film of PBSA.

Examples 4 and 7 (pullulan:HA ratio equal to 1:2), on the one hand, and 6 and 8 (pullulan:HA ratio equal to 1:3), on the other hand, allowed to verify the effect of the proportions between promoter and hyaluronic acid. In example 4, the solution obtained has optimal properties for a good electrospinning, the fibers obtained have an average diameter ranging from 800 nm to 1 µm. For the composition of example 4, which has an acid pH, the pH was increased up to 5.5 (by adding NaOH 1M) thus obtaining the solution of example 7. With the latter, the electrospinning gave regular and uniform fibers with an average diameter smaller than example 4, between 500 and 700 nm.

By increasing the proportion of hyaluronic acid, in example 6 (with acid pH) fibers with a uniform diameter were obtained, with an average value equal to 1-3 µm, while in example 8 (with pH 5.5) the fibers obtained have a non-uniform diameter ranging from 700 nm to 3 µm.

In example 5, an alginate was added to the pullulan as a stabilizer. With a promoter:HA ratio of 1:3, and under the conditions mentioned in the table, thick fibers were obtained, with an average diameter in the order of several microns.

### Preliminary in vivo evaluation of the efficiency of the composition in the cosmetic treatment of skin

A preliminary evaluation was carried out on ten volunteers, who were asked to apply a cosmetic product based on fibers obtained by electrospinning of the composition according to the invention, and a placebo. We hereby wish to clarify that the results set out below are indicative of a technical effect achieved by the composition according to the invention, which however have to be confirmed with further tests.

Each volunteer applied both the cosmetic product and also the placebo, each one on a respective arm, in particular on the palmar zone of the forearm, at the rate of one application per day for a period of four weeks.

The cosmetic product and the placebo were supplied in the form of sheets of electrospun fiber, 4x3 cm in size, on an aluminum support sheet. Both have the same base, and a cosmetic ingredient was added to the cosmetic product.

The cosmetic product used comprises hyaluronic acid HA as first compound to be electrospun, and pullulan as electrospinning promoter. The electrospun solution was prepared by dissolving 25g of hyaluronic acid and 12.5g of pullulan in 30-50mL of water, and taking its volume to 100mL by dilution, checking the pH and possibly correcting it by adding a solution of NaOH 1M so as to obtain a pH of 5.5-6.

The placebo solution, on the other hand, contains only pullulan, without hyaluronic acid. Similarly to what described for the cosmetic product, 15gr of pullulan were dissolved in 30-50 mL of water, and taken to 100mL by dilution, checking the pH and possibly correcting it by adding a solution of NaOH 1M so as to obtain a pH of 5.5-6. The solution obtained was then electrospun.

The effects of the treatment were measured using instrumental methods, in particular to assess skin hydration, skin elasticity and collagen density. The measurements were performed before the start of treatment (T0), and after 1 (T7), 2 (T14) and 4 weeks (T28) of treatment.

### Assessment of skin hydration

Skin hydration was measured with a Derma Unit SSC 3 hydration probe from Corneometer^{®}. The probe is constructed with a series of gold metal tracks to function as capacitor plates. The plates are electrically insulated by an electrical insulator, called dielectric. After the connection to an electrical power supply, electrons flow between the plates creating an electric field: the amount of charge stored by the capacitor is called capacitance. Most materials have a dielectric constant greater than vacuum, so that any material whatsoever between the capacitor plates will increase capacitance. The water present in the skin causes a change in capacity proportional to its content, giving a measurement of skin hydration in arbitrary units.

### Assessment of skin elasticity

The measurement of skin elasticity was performed with a DermaLab Combo Skinlab elasticity probe, by the company Cortex. The probe is equipped with a chamber in which a vacuum is applied, and which allows to apply a suction on the surface of the skin. The suction method includes a step of elevating and a step of retracting the skin, which are controlled by infrared sensors in the probe chamber. One of the parameters related to skin elasticity is Retraction Time, which represents the time expressed in milliseconds that is necessary for the skin to retract by 1.5 mm, after having taken it to the point of maximum elevation. Young and elastic skin quickly returns to its initial state when it is elevated, while less elastic skin will have longer retraction times. Therefore, a reduction in the retraction time is an indication of an increase in skin elasticity.

### Assessment of collagen density

High resolution images of deeper layers of the skin were obtained with a DermaLab Combo Skinlab (Cortex) ultrasound probe. The technique is based on measuring the acoustic response of the skin, when an acoustic impulse at a known frequency is sent to the skin. This acoustic impulse hits the different structures of the skin and is partly reflected. The part of the signal that is reflected is detected by an ultrasound transducer and processed in order to supply a cross-section image of the skin. The intensity of the reflected signal is indicated with a color scale, in which the darkest zones represent the zones with low response (those in which the density of the structures is low or less), while the lighter areas represent the areas with greater density. This technique allows to reconstruct the image of the skin up to a depth of 3.4 mm with a resolution of 0.06 mm, highlighting the structure of the epidermis, of the dermis and of the subcutaneous layer. An image processing software provides a value of the intensity of the echogenic response which is directly correlated to the density of the collagen. The energy of the acoustic impulses used is very low and has no negative effect on the skin and on other tissues.

### Conducting the analysis

The measurements were performed in a controlled temperature environment, at 21 ± 2°C, and with a humidity of approximately 60%.

The application of the cosmetic product and of the placebo provides to moisten the skin in correspondence with the zone to be treated, to apply the product sheet with the aluminum surface facing upward, to leave the product to act for 3 minutes on the skin, to remove the aluminum support, and eventually wait for the product to absorb completely, if necessary.

### Results

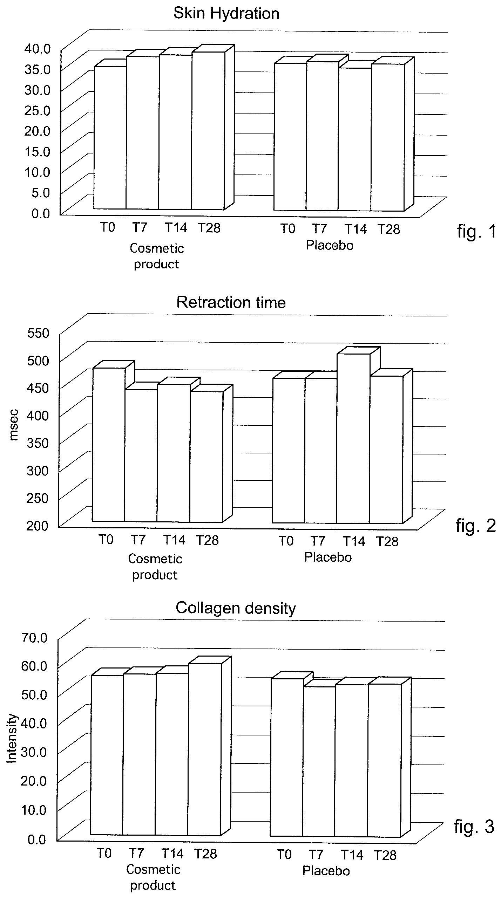

The results of the treatments in terms of skin hydration are summarized in table 1 below and in the graphs in fig. 1. The measurements of skin hydration, expressed in arbitrary corneometric units, are reported as the average value of the values recorded during the test, as the difference and percentage variation between the initial values (T0) and the values recorded at the end of the test, that is, 4 weeks (T28).

| | T0 | T7 | T14 | T28 | Difference T28-T0 | % variation T28-T0 |
|---|---|---|---|---|---|---|
| Cosmetic product | 35.0 | 37.1 | 37.5 | 38.4 | 3.4 | 9.7 |
| Placebo | 36.3 | 36.4 | 35.1 | 36.1 | -0.2 | -0.6 |

The treatment with the cosmetic product showed a significant increase in skin hydration after 28 days. The placebo treatment, on the other hand, showed no significant changes.

The results in terms of skin elasticity are summarized in table 2, and represented in the graphs of fig. 2. The elasticity data, expressed in milliseconds (msec), are reported as the average value of the values recorded during the test, as the difference and percentage variation between the initial values (T0) and the values at the end of the treatment, that is, 4 weeks (T28).

| | T0 | T7 | T14 | T28 | Difference T28-T0 | % variation T28-T0 |
|---|---|---|---|---|---|---|
| Cosmetic product | 483 | 444 | 454 | 442 | -42.0 | -8.7 |
| Placebo | 468 | 468 | 513 | 452 | -16.2 | -3.5 |

For both treatments there was a slight, but not significant, increase in skin elasticity. In the zones treated with the cosmetic product, the increase was greater than in the zones treated with the placebo, however this increase is not significant from a statistical point of view.

The results in terms of collagen density are summarized in table 3 and in the graphs of fig. 3. The collagen density data are reported as the average value of the values recorded during the test, as the difference and percentage variation between the initial values (T0) and the values at the end of the treatment, that is, 4 weeks (T28).

| | T0 | T7 | T14 | T28 | Difference T28-T0 | % variation T28-T0 |
|---|---|---|---|---|---|---|
| Cosmetic product | 56.1 | 56.1 | 56.4 | 60.3 | 4.1 | 7.4 |
| Placebo | 55.8 | 52.9 | 53.6 | 53.8 | -2.0 | -3.6 |

The treatment with the cosmetic product allowed to find an increase in collagen density, however this increase is not statistically significant. On the other hand, no increase was measured in the treatment with placebo.

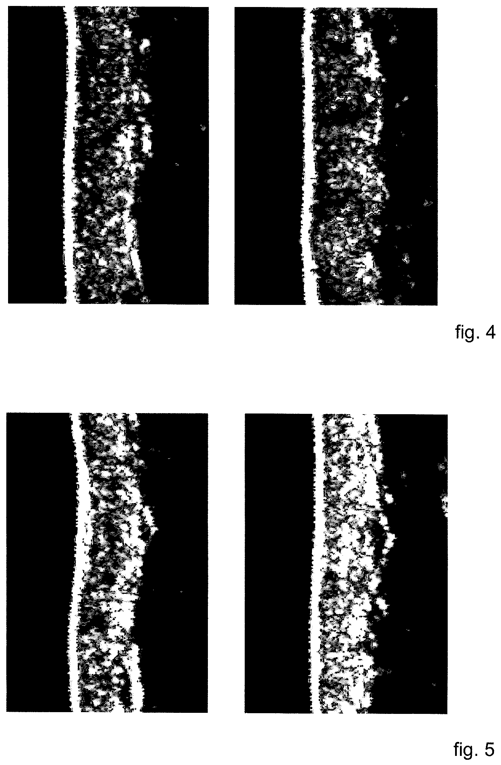

Another study was conducted to evaluate the effect of the cosmetic product on collagen density. The application modes of the cosmetic product were the same as those indicated above, but the treatment was carried out over 8 weeks. Figs. 4 and 5 represent a cross-section image of the skin, processed starting from the data collected by the instrument used, indicated above. The white zones indicate the presence of collagen. Fig. 4 shows the section of an untreated zone of the skin, at the beginning of the treatment (T0, to the left) and after 8 weeks of treatment (to the right). Fig. 5 instead shows a treated zone of the skin, at the beginning of the treatment (T0, to the left) and at the end of the treatment, after 8 weeks (to the right).

In the treated zone there is a visible increase of collagen in the skin at the end of the treatment, this increase not being found in the untreated zone.

In conclusion, the tests described above offer a preliminary evaluation of the efficiency of the cosmetic product compared to a placebo. It was possible to observe a tendency toward improvement, for all three parameters monitored, which was greater with the cosmetic product than with the placebo.

The tests carried out allowed to detect a perceptible tendency toward improvement, at least as regards collagen density.

This tendency is, among other things, confirmed by the last collagen density test, in which application times were doubled (eight weeks instead of four). The evolution of collagen density is even more evident, even if only from a visual verification. It should be emphasized that the improvement observed in the status of the collagen fibers in the skin was obtained with a single daily application of the cosmetic product, applying only hyaluronic acid, and in a shorter time than with cosmetic products of the state of the art. By way of comparison, with known products, obtaining an improvement of the collagen fibers in the skin requires longer application times (at least three months) and a greater number of daily applications.

In light of the results obtained from the preliminary study described above, it is possible to observe an interesting tendency toward improvement of the zone treated with the cosmetic product compared to the placebo, for the parameters measured, in particular for hydration and collagen density.

It is clear that modifications and/or additions of parts may be made to the composition as described heretofore, without departing from the field and scope of the present invention as defined by the claims.

It is also clear that, although the present invention has been described with reference to some specific examples, a person of skill in the art shall certainly be able to achieve many other equivalent forms of composition to be electrospun, having the characteristics as set forth in the claims and hence all coming within the scope of protection defined thereby.

## Claims

1. Composition to be electrospun, comprising a first compound to be electrospun, and an electrospinning promoter, **characterized in that** it comprises hyaluronic acid as said first compound to be electrospun and pullulan or a mixture of alginate:poly(oxyethylene) 1:1 (w/w) as said electrospinning promoter.

2. Composition as in claim 1, **characterized in that** the weight ratio between the first compound to be electrospun and the electrospinning promoter is comprised between 4:1 and 1:7.

3. Composition as in claim 1 or 2, **characterized in that** it also comprises an active ingredient.

4. Composition as in any claim hereinbefore, **characterized in that** it also comprises a stabilizer suitable to stabilize the fiber subsequently obtained.

5. Composition as in claim 4, **characterized in that** the stabilizer is a cross-linkable polymer.

6. Composition as in claim 4 or 5, **characterized in that** the electrospinning promoter is pullulan, and the stabilizer is an alginate.

7. Composition as in claim 1, **characterized in that** the promoter:hyaluronic acid weight ratio is equal to 1:3.

8. Composition as in claim 1, **characterized in that** the promoter:hyaluronic acid weight ratio is equal to 1:2.

9. Method to prepare a composition to be electrospun, comprising a step of mixing hyaluronic acid as a first compound to be electrospun with pullulan or a mixture of alginate:poly(oxyethylene) 1:1 (w/w) as an electrospinning promoter.

## Patentansprüche

1. Zusammensetzung, die elektrogesponnen werden soll, aufweisend eine erste Verbindung, die elektrogesponnen werden soll, und einen Elektrospinn-Promotor, **dadurch gekennzeichnet, dass** sie Hyaluronsäure als die erste Verbindung, die elektrogesponnen werden soll, und Pullulan oder eine Mischung aus Alginat:Poly(oxyethylen) 1:1 (w/w) als den Elektrospinn-Promotor aufweist.

2. Zusammensetzung nach Anspruch 1, **dadurch gekennzeichnet, dass** das Gewichtsverhältnis zwischen der ersten Verbindung, die elektrogesponnen werden soll, und dem Elektrospinn-Promotor zwischen 4:1 und 1:7 liegt.

3. Zusammensetzung nach Anspruch 1 oder 2, **dadurch gekennzeichnet, dass** sie außerdem einen aktiven Bestandteil aufweist.

4. Zusammensetzung nach einem der vorstehenden Ansprüche, **dadurch gekennzeichnet, dass** sie außerdem einen Stabilisator aufweist, der zur Stabilisierung der anschließend erhaltenen Faser geeignet ist.

5. Zusammensetzung nach Anspruch 4, **dadurch gekennzeichnet, dass** der Stabilisator ein vernetzbares Polymer ist.

6. Zusammensetzung nach Anspruch 4 oder 5, **dadurch gekennzeichnet, dass** der Elektrospinn-Promotor Pullulan ist und der Stabilisator ein Alginat ist.

7. Zusammensetzung nach Anspruch 1, **dadurch gekennzeichnet, dass** das Gewichtsverhältnis von Promotor zu Hyaluronsäure 1:3 beträgt.

8. Zusammensetzung nach Anspruch 1, **dadurch gekennzeichnet, dass** das Gewichtsverhältnis von Promotor zu Hyaluronsäure 1:2 beträgt.

9. Verfahren zur Herstellung einer Zusammensetzung, die elektrogesponnen werden soll, aufweisend einen Schritt des Mischens von Hyaluronsäure als eine erste Verbindung, die elektrogesponnen werden soll, mit Pullulan oder einer Mischung aus Alginat:Poly(oxyethylen) 1:1 (w/w) als ein Elektrospinn-Promotor.

## Revendications

1. Composition à électrofiler, comprenant un premier composé à électrofiler et un promoteur d'électrofilage, **caractérisée en ce qu'**elle comprend de l'acide hyaluronique en tant que dit premier composé à électrofiler et du pullulane ou un mélange d'alginate:poly(oxyéthylène) 1:1 (p/p) en tant que promoteur d'électrofilage.

2. Composition selon la revendication 1, **caractérisée en ce que** le rapport pondéral entre le premier composé à électrofiler et le promoteur d'électrofilage est compris entre 4:1 et 1:7.

3. Composition selon la revendication 1 ou 2, **caractérisée en ce qu'**elle comprend un ingrédient actif.

4. Composition selon l'une quelconque des revendications précédentes, **caractérisée en ce qu'**elle comprend également un stabilisant approprié pour stabiliser la fibre obtenue ultérieurement.

5. Composition selon la revendication 4, **caractérisée en ce que** le stabilisant est un polymère réticulable.

6. Composition selon la revendication 4 ou 5, **caractérisée en ce que** le promoteur d'électrofilage est le pullulane, et le stabilisant est un alginate.

7. Composition selon la revendication 1, **caractérisée en ce que** le rapport pondéral promoteur:acide hyaluronique est égal à 1:3.

8. Composition selon la revendication 1, **caractérisée en ce que** le rapport pondéral promoteur:acide hyaluronique est égal à 1:2.

9. Procédé de préparation d'une composition à électrofiler, comprenant une étape de mélange d'acide hyaluronique en tant que premier composé à électrofiler avec du pullulane ou un mélange d'alginate:poly(oxyéthylène) 1:1 (p/p) en tant que promoteur d'électrofilage.
